# EUROPEAN PATENT SPECIFICATION

(11) **EP 0 943 006 B1**
(45) Date of publication and mention of the grant of the patent: **13.02.2002**
(21) Application number: 97941097.4
(22) Date of filing: 19.09.1997
(51) Int. Cl.: C12P 1/02

(54) **A METHOD FOR PRODUCING AN EXTRACT FROM GANODERMA LUCIDUM CONTAINING GANODERIC ACID**
VERFAHREN ZUR HERSTELLUNG EINES GANODERICSÄURE ENTHALTENDEN EXTRAKTES AUS GANODERMA LUCIDUM
PROCEDE DE PRODUCTION D'UN EXTRAIT DE GANODERMA LUCIDUM CONTENANT DE L'ACIDE GANODERIQUE

(30) Priority: 28.09.1996 GB 9620274
(43) Date of publication of application: 22.09.1999
(73) Proprietor: Essential Nutrition Ltd., Brough HU15 1EF (GB)
(72) Inventor: WHEATLEY, Gary, William, Hull HU9 4DS (GB); CHAPMAN, Thomas, Brian, Brough HU15 1DY (GB)
(74) Representative: Stuttard, Garry Philip
(86) International application number: GB9702561
(87) International publication number: WO9813512

(56) References cited:
- PATENT ABSTRACTS OF JAPAN vol. 017, no. 124 (C-1035), 16 March 1993 & JP 04 304890 A (NITTO DENKO CORP), 28 October 1992,
- COCKS S ET AL: "High-performance liquid chromatography comparison of supercritical-fluid extraction and solvent extraction of microbial fermentation products" JOURNAL OF CHROMATOGRAPHY, vol. 697, no. 1, 21 April 1995, page 115-122 XP004023040

## Description

This invention relates to a method of production of a novel extract from Ganoderma lucidum that contains significant levels of anti-inflammatory ganoderic acids.

Ganoderma lucidum has been used for many centuries in the traditional medicines of China and Japan as a component of treatments for diseases with an inflammatory component. Recently the anti-inflammatory properties of preparations and compounds derived from Ganoderma have been confirmed by pharmacological research. Two studies are particularly worthy of note. Kohda et al, Chem. Pharm. Bull, 33(4), 1367 - 1374 - (1985) fractionated solvent extracts of Ganoderma and found significant anti-inflammatory activity using in-vitro tests such as the inhibition of histamine release from mast cells. This activity was found to be particularly associated with the compound ganoderic acid C which was considered to show promise as a non-steroidal anti-inflammatory drug (NSAID).

These findings have recently been confirmed by W B Stavinoha, Proc. 6th Int. Symp. Ganoderma Ivcidum, 3, (1995) and R T Streeper, N. Satsangi, W B Stavinoha, S T Weintraub, Supelco Reporter, 14(5), 1 - 3 (1995) who studied the pharmacological effects of an ethyl acetate extract of Ganoderma and demonstrated both topical and systemic anti-inflammatory activity by standard animal models such as the croton oil induced mouse ear inflammation tests.

Patent Abstracts of Japan 19,124 (C-1035), 16 March 1993 (JP-A-04304890)discloses the extraction of Ganoderma lucidum with conventional solvents.

Journal of Chromatography 697,1,1995, 115-122 refers to the extraction with supercritical fluid carbon dioxide of natural products from sources such as mushrooms, plants and microorganisms.

Ganoderic acids (and the related lucidenic acids) are a structurally related class of compounds based on the lanostane skeleton, based on a triterpene structure.

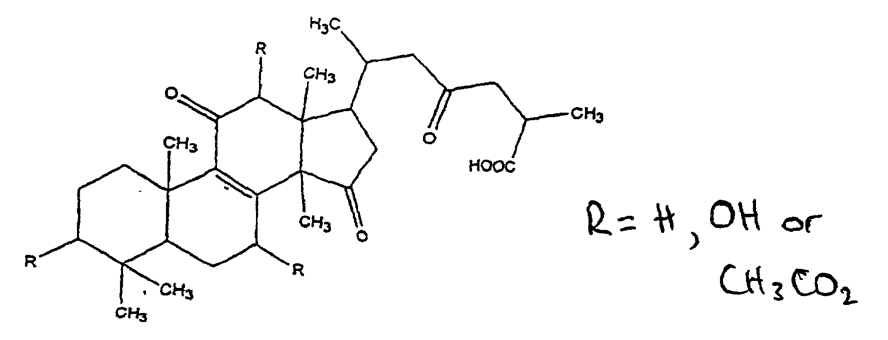

In recent years extraction the use of liquified carbon dioxide has been applied to production of fractions from natural materials rich in biologically active compounds, particularly terpenoids.

Liquid carbon dioxide has a high selectivity, being able to solubilise low molar mass non-polar compounds whilst leaving behind in the matrix higher molecular weight waxes and lipids which would otherwise increase the bulk of an extract and dilute the actives content.

Liquid carbon dioxide is superior to conventional non-polar organic solvents in that it is non-flammable so that the used solvent can be safely vented to the atmosphere, posing no waste disposal or recycling problems. The intrinsically nontoxic and highly volatile nature of carbon dioxide avoids any problems of elimination of residual levels of solvent.

According to the present invention there is provided a method of preparation of a ganoderic acid containing extract comprising steps of extraction of Ganoderma lucidum with liquid carbon dioxide and allowing the carbon dioxide to evaporate from the resultant mixture.

The resultant residual extract may be rich in ganoderic acid and may exhibit comparable anti-inflammatory activity to a conventionally obtained ethyl acetate extract from Ganoderma lucidum. However removal of residual ethyl acetate from the product is avoided.

A cosolvent may be employed, for example a polar cosolvent such as a C₁-C₄ alcohol preferably ethanol. An amount of cosolvent of 5 to 20%, preferably 20% by weight may be employed.

Preferred methods involve extraction at a pressure of 1500 to 4500 psi (100 to 310 bars) preferably about 1500 psi (100 bars) and at a temperature in the region of 20 to 35°C, preferably about 28°C.

The invention is further described by means of example but not in any limitative sense.

### Example

A quantity of roughly ground Ganoderma lucidum mushrooms were packed into a suitable pressure vessel. A volume of liquid carbon dioxide at the ratio of approximately 6 ml to 1 g of vegetable matter was allowed to pass through the raw material at a rate of approximately 1 ml/min. The liquid carbon dioxide was then collected and the pressure was released so that the carbon dioxide was allowed to vent to the atmosphere. The extract remained in the collection vessel as an oil or semi-solid depending on the exact extraction conditions.

The following extraction conditions were employed:

| SAMPLE | PRESSURE | TEMP | COSOLVENT | %YIELD |
|---|---|---|---|---|
| Gan 1 | 100 bars (1500 psi) | 28°C | None | 0.3% |
| Gan 2 | 100 bars (1500 psi) | 28°C | 10% Ethanol | 1.8% |
| Gan 3 | 310 bars (4500 psi) | 50°C | None | 0.6% |
| Gan 4 | 310 bars (4500 psi) | 50°C | 10% Ethanol | N/A |

The chemical composition of the extracts was studied using the TLC method published by Kohda et al.

Ganoderic acid B and Ganoderic acid C were identified in all extracts by the characteristic red spots after treatment with a ceric sulphate reagent with Rfs of approximately 0.25 and 0.42 as described by Kohda et al. These components were further found to exhibit a characteristic fluorescence when exposed to UV light of 254 nm.

An ethyl extract of Ganoderma lucidum was prepared for purposes of comparison. The TLC profile of the liquid carbon dioxide extracts was essentially identical to that of the ethyl acetate extract, and the extracts contained comparable levels of ganoderic acids B & C.

## Claims

1. A method of preparation of a ganoderic acid containing extract comprising the steps of extraction of Ganoderma lucidum with liquid carbon dioxide and allowing the carbon dioxide to evaporate from the resultant mixture.

2. A method as claimed in claim 1, wherein the extraction is carried out with a mixture of liquid carbon dioxide and a polar cosolvent.

3. A method as claimed in claim 2, wherein the cosolvent is a C₁ - C₄ alcohol.

4. A method as claimed in claim 3, wherein the cosolvent is ethanol.

5. A method as claimed in any of claims 2 to 4, wherein the amount of cosolvent is 5 to 20% by weight.

6. A method as claimed in claim 5, wherein the amount of cosolvent is 20% by weight.

7. A method as claimed in any preceding claim, wherein the extraction is carried out at a pressure of 100 to 310 bars (1500 to 4500 psi).

8. A method as claimed in claim 7, wherein the extraction is carried out at a pressure of 100 bars (1500 psi).

9. A method as claimed in any preceding claim, wherein the extraction is carried out at a temperature of 20 to 35°C.

10. A method as claimed in claim 9, wherein the temperature is 20°C.

## Patentansprüche

1. Verfahren zur Herstellung von Ganodersäure enthaltendem Extrakt, mit den Schritten der Extraktion von Ganoderma Lucidum mit flüssigem Kohlendioxid und des Verdampfenlassens des Kohlendioxids aus der resultierenden Mischung.

2. Verfahren nach Anspruch 1, bei dem die Extraktion mit einer Mischung aus flüssigem Kohlendioxid und einem polaren zusätzlichen Lösungsmittel durchgeführt wird.

3. Verfahren nach Anspruch 2, bei dem das zusätzliche Lösungsmittel ein C₁- bis C₄-Alkohol ist.

4. Verfahren nach Anspruch 3, bei dem das zusätzliche Lösungsmittel Ethanol ist.

5. Verfahren nach einem der Ansprüche 2 bis 4, bei dem die Menge an zusätzliche Lösungsmittel 5 bis 20 Gew.% beträgt.

6. Verfahren nach Anspruch 5, bei dem die Menge an zusätzliche Lösungsmittel 20 Gew.% beträgt.

7. Verfahren nach einem der vorhergehenden Ansprüche, bei dem die Extraktion bei einem Druck von 100 bis 310 bar (1500 bis 4500 psi) durchgeführt wird.

8. Verfahren nach Anspruch 7, bei dem die Extraktion bei einem Druck von 100 bar (1500 psi) durchgeführt wird.

9. Verfahren nach einem der vorhergehenden Ansprüche, bei dem die Extraktion bei einer Temperatur von 20 bis 35 °C durchgeführt wird.

10. Verfahren nach Anspruch 9, bei dem die Temperatur 20 °C beträgt.

## Revendications

1. Procédé de préparation d'un acide ganodérique contenant un extrait, comprenant les étapes consistant à extraire *Ganoderma lucidum* avec du dioxyde de carbone liquide et à laisser le dioxyde de carbone s'évaporer du mélange obtenu.

2. Procédé selon la revendication 1, dans laquelle l'extraction est réalisée avec un mélange de dioxyde de carbone liquide et un cosolvant polaire.

3. Procédé selon la revendication 2, dans laquelle le cosolvant est un alcool en C₁ à C₄.

4. Procédé selon la revendication 3, dans laquelle le cosolvant est l'éthanol.

5. Procédé selon l'une quelconque des revendications 2 à 4, dans laquelle la quantité de cosolvant est comprise entre 5 et 20 % en poids.

6. Procédé selon la revendication 5, dans laquelle la quantité de cosolvant est de 20 % en poids.

7. Procédé selon l'une quelconque des revendications précédentes, dans laquelle l'extraction est réalisée à une pression comprise entre 100 et 310 bars (1500 et 4500 psi).

8. Procédé selon la revendication 7, dans laquelle l'extraction est réalisée à une pression de 100 bars (1500 psi).

9. Procédé selon l'une quelconque des revendications précédentes, dans laquelle l'extraction est réalisée à une température comprise entre 20 et 35°C.

10. Procédé selon la revendication 9, dans laquelle la température est de 20°C.
